(19) Europäisches Patentamt
European Patent Office
Office européen des brevets

(11) EP 1 623 972 A1

(12) **EUROPEAN PATENT APPLICATION**
published in accordance with Art. 158(3) EPC

(43) Date of publication:
**08.02.2006 Bulletin 2006/06**

(21) Application number: **04730033.0**

(22) Date of filing: **28.04.2004**

(51) Int Cl.:
***C07C 233/47*** *(1990.01)* ***A61K 8/30*** *(0000.00)*

(86) International application number:
**PCT/JP2004/006114**

(87) International publication number:
**WO 2004/099121 (18.11.2004 Gazette 2004/47)**

(84) Designated Contracting States:
**DE FR**

(30) Priority: **07.05.2003 JP 2003129626**

(71) Applicant: **Ajinomoto Co., Inc.**
**Tokyo 104-8315 (JP)**

(72) Inventors:
- **TOBITA, Kazuhiko,**
**c/o Ajinomoto Co., Inc.**
**Kawasaki-shi,**
**Kanagawa 2108681 (JP)**
- **YAMAGUCHI, Yoshinori,**
**c/o Ajinomoto Co., Inc.**
**Kawasaki-shi,**
**Kanagawa 2108681 (JP)**
- **YAMATO, Naoya,**
**c/o Ajinomoto Co., Inc.**
**Kawasaki-shi,**
**Kanagawa 2108681 (JP)**

(74) Representative: **Hart Davis, Jason et al**
**Cabinet Beau de Loménie,**
**158, rue de l'Université**
**75340 Paris Cedex 07 (FR)**

(54) **SOLID N-ACYLALANINE OR SALTS THEREOF**

(57) The present invention relates to
solid N-acylalanine comprising one kind or two or more kinds of compounds, each having an acyl group having a different number of carbon atoms, which is/are selected from the compounds represented by the formula (I):

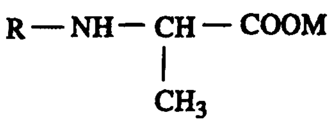

wherein R is a straight chain or branched chain, saturated or unsaturated acyl group having 8 to 22 carbon atoms, and M is a hydrogen atom, an alkali metal, an alkaline earth metal, ammonium, an organic amine or a basic amino acid, or a salt thereof, and
a cleansing composition comprising the solid N-acylalanine or a salt thereof.

The solid acylalanine or a salt thereof of the present invention is superior in solubility, and is free of moisture absorption or caking during preservation and agglomeration on dissolution in water. A powder or granular cleansing composition containing the solid N-acylalanine or a salt thereof as a main component (main cleansing component) achieves a cleansing agent having extremely high property, which is free of caking during preservation and coagulation in practical use, superior in the basic performance of cleansing agents (lather characteristics), free of slimyness and friction during use, and safe with less irritation.

EP 1 623 972 A1

## Description

### Technical Field

The present invention relates to a solid acylalanine or a salt thereof, which can be easily solidified, is superior in producibility and solubility, and is free of moisture absorption or caking during preservation and agglomeration on dissolution in water, as well as a powder or granular cleansing composition using the solid acylalanine or a salt thereof, which is free of caking due to moisture absorption during preservation and agglomeration of powder in practical use, and which is quick in lathering, superior in the basic performance of cleansing agents such as lather amount and lather retention, free of slimyness and friction during use, and safe with less irritation, and a cleansing agent for skin and hair, which contains the same.

### Background Art

Conventionally, as a main starting material of cleansing components such as solid soap, facial foam and facial powder, various solid cleansing agents have been used. While solid substances such as fatty acid soap, alkyl sulfate, alkyl phosphate, N-acyl isethionate and the like have been used due to their high lather amount and high cleansing performance, they are defective in that they induce irritation and are inferior in the sense of use.

While N-acyl glutamate salt is known to a low irritant anionic surfactant, a solid N-acyl glutamate salt (solid substance of N-acyl glutamate salt) is associated with problems in that it is inferior in foamability and gives a sense of slimyness after use.

N-acylalanine salt was introduced as a highly safe cleansing agent having selective cleansing function in JP-A-2000-265191, N-acylalanine salt was introduced as causing less irritation to the skin and/or hair and being superior in lather retention, lather quality and sense of use in JP-A-8-3585 and JP-A-8-60189, thereby establishing the high performance thereof as a cleansing agent. In JP-A-8-3585, JP-A-8-60189 etc., for example, it is used as a cleansing component of liquid shampoo, liquid body shampoo and the like.

However, there is no report relating to N-acylalanine salt in a solid form, and JP-A-2002-338994 solely shows a powder or a granular cleansing agent using N-cocoylalanine Na. However, the powder or granular cleansing agent described in this publication is a powdered or granulated composition comprising N-cocoylalanine Na and at least cationic dextran, and does not suggest the presence of a powder or granule (solid substance) comprising only acylalanine or a salt thereof. According to the study of the present inventors, moreover, a powder or granule of this composition has high moisture absorbability, not entirely superior solubility in water, only insufficiently expressed basic performance (foamability, lather amount, lather retention etc.) of N-cocoylalanine Na as a cleansing agent. Thus, they are not satisfactory as solid cleansing agents.

In general, powder or granular cleansing agents easily become a cake due to moisture absorption of water or addition of a liquid starting material and allow limited addition of surfactant, other component and the like. Thus, it is particularly difficult to simultaneously achieve superior preservation stability and superior foamability and usability (sense of use). However, since powder or granular cleansing agents do not substantially contain water, they can advantageously prepare a highly safe product free of preservative and antimicrobial agent. Thus, the development of a powder or granular cleansing agent superior in preservation stability and also superior in foamability and usability (sense of use) is strongly demanded.

Addition of an anionic surfactant to achieve superior foamability of a powder or granular cleansing agent is known. In addition, of the anionic surfactants, alkyl sulfate, alkyl phosphate etc. are known to have particularly superior lathering property. However, due to the irritation they induce, cleansing agents containing them are inferior in the sense of use. Furthermore, addition of a fatty acid salt to a powder or granular cleansing agent for improving the foamability is also known. Fatty acid salts have superior lathering property, but fatty acid salt itself is known to induce a frictional feeling, dry skin and the like.

On the other hand, JP-A-2002-338994 proposes, as a powder cleansing composition using a low irritant anionic surfactant, a powder cleansing composition containing N-lauroyl glutamate salt. However a cleansing agent containing N-lauroyl glutamate salt shows poor lathering and tends to show slimyness after use. JP-A-2001-26524 proposes a powder cleansing composition containing N-acyl glutamate salt and N-acyl isethionate salt. While this publication describes that a concurrent use of N-acyl glutamate salt, which is low foamable and slimy during use, and N-acyl isethionate salt resulted in improved lathering property and slimyness, in the study of the present inventors, such effect was afforded only at an extremely limited amount ratio (weight ratio) of use, and the effect was not sufficient.

In view of the above-mentioned situation, the present invention aims at providing a powder or granular cleansing composition which is free of caking due to moisture absorption during preservation and agglomeration of powder in practical use, and which is quick in lathering, superior in the basic performance of cleansing agents such as lather amount and lather retention, free of slimyness and friction during use, and safe with less irritation, and a cleansing agent

for skin and hair containing the same, and furthermore, solid N-acylalanine or a salt thereof superior in solubility, which in itself is free of caking due to moisture absorption during preservation and agglomeration on dissolution in water, and which can achieve such powder or granular cleansing composition having superior properties.

## Disclosure of the Invention

The present inventors have conducted intensive studies in an attempt to achieve the above-mentioned objects and found that N-acylalanine or a salt thereof can be obtained in a solid state from an aqueous solution, an organic solvent solution and the like of N-acylalanine or a salt thereof by controlling the standard deviation of acyl group chain length distribution to not more than a particular value according to the kind of the salt. Furthermore, they have found that granule or powder cleansing agents containing the same show superior preservation stability and solubility in water in practical use, and improved basic performance of cleansing agents such as quick lathering, lather amount and the like, as well as reduced slimyness and friction during use. Moreover, they have found that, as compared to other salts, dispersion in the acyl group chain length distribution can be tolerated in basic amino acid salt from among the N-acylalanine salts, which in turn facilitates powderization, which resulted in the completion of the present invention.
Accordingly, the present invention relates to

(1) solid N-acylalanine comprising one kind or two or more kinds of compounds, each having an acyl group having a different number of carbon atoms, which is/are selected from the compounds represented by the formula (I):

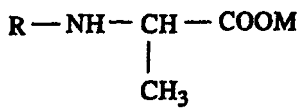

wherein R is a straight chain or branched chain, saturated or unsaturated acyl group having 8 to 22 carbon atoms, and M is a hydrogen atom, an alkali metal, an alkaline earth metal, ammonium, an organic amine or a basic amino acid, or a salt thereof,
(2) solid N-acylalanine of the above-mentioned (1), which comprises a long chain fatty acid or a salt thereof, or a salt thereof,
(3) solid N-acylalanine comprising one kind or two or more kinds of compounds, each having an acyl group having a different number of carbon atoms, which is/are selected from the compounds represented by the formula (I):

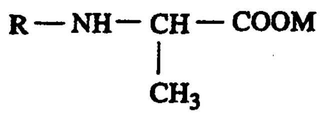

wherein R is a straight chain or branched chain, saturated or unsaturated acyl group having 8 to 22 carbon atoms, and M is a hydrogen atom, an alkali metal, an alkaline earth metal, ammonium or an organic amine,
which shows the standard deviation ($\sigma$) of the acyl group chain length distribution of not more than 2.0,
or a salt thereof,
(4) the solid N-acylalanine of the above-mentioned (3), wherein M in the formula (I) is an alkali metal, or a salt thereof,
(5) the solid N-acylalanine of the above-mentioned (4), wherein the alkali metal is potassium, or a salt thereof,
(6) the solid N-acylalanine of any one of the above-mentioned (3) - (5), which comprises a long chain fatty acid or a salt thereof, or a salt thereof,
(7) solid N-acylalanine comprising one kind or two or more kinds of compounds, each having an acyl group having a different number of carbon atoms, which is/are selected from the compounds represented by the formula (I):

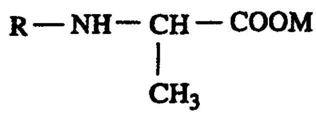

wherein R is a straight chain or branched chain, saturated or unsaturated acyl group having 8 to 22 carbon atoms, and M is a basic amino acid,

which shows the standard deviation (σ) of the acyl group chain length distribution of not more than 5.0, or a salt thereof,

(8) the solid N-acylalanine of the above-mentioned (7), which comprises a long chain fatty acid or a salt thereof, or a salt thereof,

(9) the solid N-acylalanine of the above-mentioned (7) or (8), wherein the basic amino acid for M in the formula (I) is lysine or arginine, or a salt thereof,

(10) the solid N-acylalanine of any one of the above-mentioned (1) - (9), which is used for a powder or granular cleansing agent, or a salt thereof,

(11) a powder or granular cleansing composition comprising at least (A) the solid N-acylalanine of any one of the above-mentioned (1) - (9) or a salt thereof,

(12) the powder or granular cleansing composition of the above-mentioned (11), further comprising (B) an inorganic or organic powder,

(13) the powder or granular cleansing composition of the above-mentioned (11) or (12), further comprising (C) acylisethionic acid or a salt thereof and/or (D) acylamino acid or a salt thereof (excluding the N-acylalanine or a salt thereof for the aforementioned (A)), and

(14) a cleansing agent for skin or hair, comprising the powder or granular cleansing composition of any one of the above-mentioned (11) - (13).

**Best Mode for Embodying the Invention**

The present invention is explained in detail in the following.

The solid N-acylalanine or a salt thereof of the present invention is a solid substance comprising one kind or two or more kinds of compounds, each having an acyl group having a different number of carbon atoms, which is/are selected from the compounds represented by the formula (I):

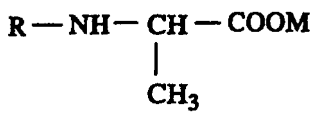

wherein R is a straight chain or branched chain, saturated or unsaturated acyl group having 8 to 22 carbon atoms, and M is a hydrogen atom, an alkali metal, an alkaline earth metal, ammonium, an organic amine or a basic amino acid, and preferably a solid substance having a standard deviation (σ) of acyl group chain length distribution of not more than 2.0.

As used herein, the standard deviation (σ) of acyl group chain length distribution is calculated by the following formula (II):

$$\sigma = h*((1/(N-1))*(\sum U^2F-(\sum UF)^2/N))(1/2) \qquad \text{(II)}$$

wherein h is a difference in the carbon atom number of the adjacent acyl group chain lengths, which is 2 in the present invention, U is $(X-X_0)/h$ wherein $X_0$ is a typical value of the carbon atom number of acyl group chain length (i.e., 12, which is the carbon atom number of lauroyl group), and X is a carbon atom number of the acyl group chain length of N-acylalanine or a salt thereof (single compound) comprising any one kind of compound selected from the compounds represented by the formula (I), or N-acylalanine or a salt thereof (mixture) comprising two or more kinds of compounds selected from the compounds represented by the formula (I), F is a wt% thereof, and N is a total of F.

In other words, the solid N-acylalanine or a salt thereof of the present invention is obtained by solidification of N-acylalanine or a salt thereof obtained by acylation of amino group of α-alanine (D-alanine, L-alanine or DL-alanine, or a mixture of two or more kinds selected from these) with fatty acid, preferably one obtained by solidification of basic amino acid salt of N-acylalanine having a standard deviation (σ) of acyl group chain length distribution of not more than 2.0.

The solid N-acylalanine or a salt thereof of the present invention can be obtained from, for example, an aqueous solution, an organic solvent solution or a mixed solvent (water+organic solvent) solution of N-acylalanine or a salt thereof by solvent removal operation under the conditions of heating, reduced pressure and the like, or cooling crystal precipitation and the like. While N-acylalanine is generally synthesized by adding fatty acid chloride to an alanine alkali solution, N-acylalanine having a standard deviation (σ) of acyl group chain length distribution of not more than 2.0 can be obtained by the use of, as a fatty acid chloride to be used therefor, a fatty acid chloride having a standard deviation (σ) of fatty acid chain length distribution of not more than 2.0, and by neutralization of the thus-obtained N-acylalanine having a

standard deviation (σ) of fatty acid chain length distribution of not more than 2.0 with a base, N-acylalanine salt having a standard deviation (σ) of fatty acid chain length distribution of not more than 2.0 can be obtained. For example, the distribution of the acyl group chain length of N-acylalanine or a salt thereof can be measured using high performance liquid chromatography (HPLC) and calculated from a peak area ratio of N-acylalanine of each chain length.

The solid N-acylalanine and a salt thereof of the present invention can be used as a cleansing component of various hair cleansing agents such as shampoo, conditioning shampoo and the like, various skin cleansing agents such as face cleansing agents (facial foam, facial powder etc.), makeup removing agents, cleansing cream, body shampoo, hand soap, solid soap, shaving foam, shaving cream and the like, toothpaste and the like, and particularly preferably as a cleansing component having high cleansing component concentration such as solid soap, facial foam, powder or granular cleansing agents (facial powder etc.) and the like, particularly preferably as a cleansing component of powder or granular cleansing agents.

When the solid N-acylalanine or a salt thereof of the present invention is N-acylalanine or N-acylalanine salt of the formula (I) wherein M is a hydrogen atom, an alkali metal, an alkaline earth metal, an ammonium or an organic amine, it is important that the standard deviation (σ) of fatty acid chain length distribution be not more than 2.0. When the standard deviation (σ) of fatty acid chain length distribution exceeds 2.0, solidification thereof is not easy, and even if solidification is possible, the resulting solid substance has high moisture absorbability, sometimes becomes solidified during preservation, and shows low solubility in water. When the solid substance is futher processed into a powder or granule, it may fail to remain a powder or granule due to moisture absorption during preservation or show low dissolution rate in water. Furthermore, a powder or granular cleansing composition prepared using such a solid substance agglomerates, is slow in lathering, and is insufficient in lather amount and lather retention. Thus, when the solid N-acylalanine or a salt thereof of the present invention is one of the formula (I) wherein M is a hydrogen atom, an alkali metal, an alkaline earth metal, an ammonium or an organic amine, the standard deviation (σ) of acyl group chain length distribution is more preferably not more than 1.7, still more preferably not more than 1.1, particularly preferably not more than 0.3.

Furthermore, when the solid N-acylalanine or a salt thereof of the present invention is one of the formula (I) wherein M is a basic amino acid salt comprising basic amino acid, the melting point of the powder becomes high as compared to other salts such as alkali metal salt and the like, and solidification (drying) becomes easy, which in turn makes it possible to obtain a powder (granule) more easily at a high recovery rate. Therefore, when the standard deviation (σ) of acyl group chain length distribution is not more than 5.0, solidification is possible, the obtained solid substance (powder or granule) resists moisture absorption and shows solubility in water in practical use, and the basic performance of cleansing agents, such as lathering, lather amount and the like, become fine. Such basic amino acid salt of N-acylalanine having a standard deviation (σ) of acyl group chain length distribution of not more than 5.0 can be obtained by, in the same manner as the above-mentioned, using fatty acid chloride having a standard deviation (σ) of fatty acid chain length distribution of not more than 5.0 as fatty acid chloride for acylation of alanine, and neutralization of the thus-obtained N-acylalanine having a standard deviation (σ) of acyl group chain length distribution of not more than 5.0 with a basic amino acid.

In such basic amino acid salt of solid N-acylalanine, the standard deviation (σ) of acyl group chain length distribution is more preferably not more than 4.0, more preferably not more than 3.3, still more preferably not more than 2.7, yet more preferably not more than 2.0, particularly preferably not more than 1.7 and most preferably not more than 1.1.

In the present invention, the range of the "acyl group chain length distribution" where a solid acylalanine salt can be obtained more stably can be determined by refining using a counter salt (basic component).

The "solid" of the solid N-acylalanine or a salt thereof of the present invention means that N-acylalanine or a salt thereof is in a state of substance capable of showing a definite melting point. In the solid N-acylalanine or a salt thereof of the present invention shows, for example, a melting point within the range of 75 - 95°C when the salt is an alkali metal salt, and a melting point of not less than 200°C when the salt is a basic amino acid salt.

In the solid N-acylalanine or a salt thereof of the present invention, it is important that R in the formula (I) have 8 to 22 carbon atoms. When N-acylalanine or a salt thereof having an acyl group chain length of less than 8 is contained, foamability and the sense of use may be degraded, and since cleansing agents (particularly, powder or granular cleansing agents) containing such solid substance as a cleansing component show degraded lathering property and give a frictional feeling, the sense of use is also degraded. On the other hand, when N-acylalanine (salt) having an acyl group chain length of more than 22 is contained, foamability and the sense of use may be degraded, and since cleansing agents (particularly, powder or granular cleansing agents) containing such solid substance as a cleansing component show degraded lathering property and give slyminess, the sense of use is also degraded.

In the solid N-acylalanine or a salt thereof of the present invention, R in the formula (I) preferably has a carbon atom number of 10 to 18 and particularly preferably a carbon atom number of 12 to 16. Specific examples of R (acyl group) include 2-ethylhexanoyl group, capryloyl group, caproyl group, lauroyl group, myristoyl group, palmitoyl group, stearoyl group, isostearoyl group, oleoyl group, behenoyl group and the like. Of these, caproyl group, lauroyl group, myristoyl group, palmitoyl group are preferable.

When the solid N-acylalanine or a salt thereof of the present invention comprises two or more kinds of compounds, each having an acyl group having a different number of carbon atoms, which are selected from the compounds represented by the formula (I), not less than 50 wt%, preferably not less than 60 wt%, particularly preferably not less than 80 wt%, of the whole preferably consists of particular one kind of N-acylalanine (salt).

Here, as the "particular one kind of N-acylalanine (salt)", one represented by the formula (I) wherein R is a caproyl group, a lauroyl group, a myristoyl group or a palmitoyl group is preferable, and one wherein R is a lauroyl group is particularly preferable.

The solid N-acylalanine or a salt thereof of the present invention may consist of unneutralized N-acylalanine alone, but it preferably consists of unneutralized N-acylalanine and a salt, or a salt alone. When it consists of unneutralized N-acylalanine and a salt, a neutralization equivalent amount is preferably 0.80 - 1.10, more preferably 0.85 - 1.05, particularly preferably 0.90 - 1.00. Alternatively, it may be D-form alone, L-form alone, DL-form alone, or a mixture of two or more kinds selected therefrom.

As the basic component (i.e., M in the formula (I)) in the case of a salt, alkali metal, alkaline earth metal, ammonium, organic amines and basic amino acid can be mentioned. As the alkali metal, potassium is preferable, as the alkaline earth metal, magnesium, calcium and the like are preferable, as the organic amine, monoethanolamine, diethanolamine, triethanolamine, 2-amino-2-methyl-1-propanol, 2-amino-2-methyl-1,3-propanediol and the like are preferable, and as the basic amino acid, lysine, ornithine, arginine and the like are preferable. From the aspects of easy production and low moisture absorbability, alkali metal salt and basic amino acid salt are preferable, and potassium salt, arginine salt and lysine salt are more preferable. In addition, since the melting point of a solid substance is high and a high recovery rate can be achieved after drying, basic amino acid salts are preferable. Of the basic amino acid salts, arginine salt is preferable since it has less odor and is used for facial wash and the like.

The solid N-acylalanine or a salt thereof of the present invention is not particularly limited by its form as long as it is in a solid state. Specifically, for example, powder, granule, flake and the like can be mentioned. In addition, the solid N-acylalanine or a salt thereof of the present invention is preferably solid N-acylalanine or a salt thereof, which contains long chain fatty acid or a salt thereof. Here, the "solid N-acylalanine or a salt thereof, which comprises long chain fatty acid or a salt thereof" means a solid substance prepared by forming a solution (e.g., aqueous solution etc.) of N-acylalanine (salt) and long chain fatty acid (salt) and solvent removal or crystal precipitation from the solution to eutectic crystal of N-acylalanine (salt) and long chain fatty acid (salt). Alternatively, it means a solid state mixture obtained by mechanically mixing solid N-acylalanine or a salt thereof and solid long chain fatty acid or a salt thereof. The solid N-acylalanine or a salt thereof of the present invention processed into a solid substance containing long chain fatty acid or a salt thereof as mentioned above are futher improved in solubility in water, as well as workability during solidification and yield. In the present invention, the solid N-acylalanine or a salt thereof, which comprises long chain fatty acid or a salt thereof, is preferably a solid substance comprising eutectic crystal of N-acylalanine (salt) and long chain fatty acid (salt).

The above-mentioned long chain fatty acid or a salt thereof is a salt of a straight chain or branched chain, saturated or unsaturated fatty acid, which may be single compounds having the same carbon atom number, or a mixture of compounds having different carbon atom numbers. The carbon atom number in the case of single compounds having the same carbon atom number, or a mixture of compounds having different carbon atom numbers is 8 - 22, preferably 8 - 18. As the basic component of the salt, alkali metals such as sodium, potassium and the like, alkaline earth metals such as magnesium, calcium and the like, organic amines such as monoethanolamine, diethanolamine, triethanolamine, 2-amino-2-methyl-1-propanol, 2-amino-2-methyl-1,3-propanediol and the like, inorganic amines such as ammonia and the like, basic amino acids such as lysine, ornithine, arginine and the like, and the like can be mentioned, with preference given to alkali metal or basic amino acid.

Specific examples of the long chain fatty acid or a salt thereof include caprylic acid salt, capric acid, lauric acid, myristic acid, palmitic acid, stearic acid, coconut oil fatty acid, hydrogenated tallow fatty acid, palm oil fatty acid, hydrogenated palm oil fatty acid, safflower oil fatty acid, tallow fatty acid, behenic acid, oleic acid, isostearic acid or salts thereof can be mentioned. Of these, capric acid, lauric acid, myristic acid salt and palmitic acid salt are preferable.

The long chain fatty acid or a salt thereof may be used alone or two or more kinds thereof may be used concurrently. While the amount thereof to be added is not particularly limited, it is generally preferably 1 - 20 wt%, more preferably 2 - 12 wt%, still more preferably 3 - 10 wt%, particularly preferably 5 - 10 wt%, of the N-acylalanine or a salt thereof, in view of release property after drying for solidification of solid N-acylalanine or a salt thereof and solubility of the solid substance in water. When the content of long chain fatty acid or a salt thereof is less than 1%, the solubility may be degraded, thus preventing lathering. The content of long chain fatty acid or a salt thereof of more than 20% is not preferable, since moisture absorbability of the powder becomes high, thereby causing caking and the like.

While the moisture content of the solid N-acylalanine or a salt thereof of the present invention is not particularly limited, it is not more than 8 wt%, more preferably not more than 5 wt%, particularly preferably not more than 2 wt%, from the viewpoints of caking during preservation and solubility in water.

The powder or granular cleansing composition of the present invention is characterized in that at least the above-mentioned solid N-acylalanine or a salt thereof of the present invention (=(A)) is contained, which in turn affords

a powder or granular cleansing composition superior in stability during preservation, as well as solubility and (the sense of) usability. Generally, a powder cleansing composition is prepared by adding at least an inorganic powder or an organic powder, which becomes a builder, besides the cleansing agents (cleansing components) and pulverizing the mixture. In addition, a granular cleansing composition is prepared by granulating, under optimal conditions, the mixture after the aforementioned mixing and pulverizing.

The powder or granular cleansing composition of the present invention preferably further comprises (B) an inorganic or organic powder in addition to the above-mentioned solid N-acylalanine or a salt thereof of the present invention (=(A)), from the viewpoints of anti agglomeration in practical use and the like. The inorganic or organic powder is not particularly limited as long as it is insoluble in water or has low moisture absorbability and, for example, organic powders such as nylon powder, polyethylene powder, polymethylmethacrylate powder, polystyrene powder, styrene·acrylic acid copolymer resin powder, benzoguanamine resin powder, polytetrafluoroethylene powder, cellulose powder, cornstarch, hydroxypropylstarchphosphoric acid, lauroyl lysine and the like, inorganic powders such as trimethylsilsesquioxane powder, silicone resin powder, talc, kaolin, mica, sericite, white mica, gold mica, synthesis mica, red mica, lithia mica, vermiculite, barium sulfate, silica, zeolite, sintered calcium sulfate, apatite fluoroxide, hydroxy apatite, ceramic powder, zinc myristate, calcium palmitate, aluminum stearate, boron nitride, titanium dioxide, zinc oxide, iron oxide red, iron titanate, titanium oxide fine particle, needle-like titanium oxide, spindle titanium oxide, rod-like titanium oxide, zinc oxide fine particle, thin section zinc oxide and the like, and the like can be mentioned. Of these, nylon powder, polyethylene powder, polystyrene powder, cellulose powder, cornstarch, hydroxypropylstarchphosphoric acid, lauroyl lysine, silicone resin powder, talc, silica, titanium dioxide and fine titanium oxide are preferable.

The inorganic or organic powder for ingredient (B) may be used alone or two or more kinds thereof may be used concurrently. While the amount thereof to be added is not particularly limited, it is preferably 10 - 70 wt%, more preferably 20-50 wt%, of the whole composition. The amount of B component to be added is less than 10 wt%, the effect of improving anti-agglomeration property in practical use is difficult to achieve. When the amount of B component to be added is greater than 70 wt%, the amount of the cleansing component becomes smaller, sufficient lathering property cannot be obtained, and the sense of use is degraded. To maintain fine sense of use such as feel, lather amount and the like, and from the viewpoints of anti-agglomeration property and the like, 10 - 70 wt% is preferable.

The powder or granular cleansing composition of the present invention preferably comprises the above-mentioned solid N-acylalanine or a salt thereof of the present invention (=(A)) and further (C) acyl isethionic acid or a salt thereof and/or (D) acylamino acid or a salt thereof (excluding N-acylalanine or a salt thereof for the aforementioned (A)), from the viewpoints of lathering performance such as lather retention and the like.

The ingredient (C) acyl isethionic acid or a salt thereof may be entirely a salt, or the entire portion may be used in the form of an unneutralized acid, or used after adjusting to any neutralization equivalent amount. As the acyl group, for example, 2-ethylhexanoyl group, capryloyl group; caproyl group, lauroyl group, myristoyl group, palmitoyl group, stearoyl group, isostearoyl group, oleoyl group, behenoyl group, cocoyl group, tallow fatty acyl group, hydrogenated tallow fatty acyl group, palm oil fatty acyl group and the like can be mentioned. As the salt, metal salts such as sodium, potassium and the like, ammonium salt, ethanolamine salt and the like can be mentioned. From the viewpoints of foamability, as preferable acyl isethionic acid or a salt thereof, coconut oil fatty acid ethyl ester sodium sulfonate, ethyl laurate sodium sulfonate etc. or salts of these can be mentioned.

Acyl isethionic acid or a salt thereof for ingredient (C) may be used alone or two or more kinds thereof may be used concurrently. While the amount thereof to be added is not particularly limited, the weight ratio (C/A) to ingredient (A) is preferably 1/99-99/1, more preferably 10/90-90/10. When the amount of ingredient (C) is small and apart from the weight ratio, lather retention cannot be sufficiently improved and when the amount of ingredient (C) is high, sufficient expression of superior feel (sense of use) by ingredient (A) becomes unpreferably difficult. Thus, C/A is preferably 1/99-99/1 from the viewpoints of further improvement of lathering performance such as lather retention and the like, while maintaining a superior feel (sense of use) by ingredient (A).

The N-acylamino acid or a salt thereof (excluding N-acylalanine or a salt thereof for the aforementioned (A)) for ingredient (D) may be a salt of acylamino acid comprising single compounds having the same acyl group, or a salt of acylamino acid comprising a mixture of compounds having different acyl groups. While amino acid is not particularly limited, glutamic acid, glycine, threonine, sarcosine, N-methyl-β-alanine and the like are preferable (namely, N-acyl glutamate or a salt thereof, N-acylglycine or a salt thereof, N-acylthreonine or a salt thereof, N-acylsarcosine or a salt thereof, N-methyl-β-alanine or a salt thereof and the like are preferable). These may be optically active forms, any mixture of optical antipodes or racemates. The acyl group is a straight chain or branched chain, saturated or unsaturated acyl group and, for example, 2-ethylhexanoyl group, capryloyl group, caproyl group, lauroyl group, myristoyl group, palmitoyl group, stearoyl group, isostearoyl group, oleoyl group, behenoyl group, cocoyl group, tallow fatty acyl group, hydrogenated tallow fatty acyl group, palm oil fatty acyl group and the like can be mentioned. The carbon atom number of acyl group when N-acylamino acid or a salt thereof is a single compound is preferably 8 - 22, particularly preferably 10 - 18, most preferably 12 - 16. The average carbon atom number is preferably 8 - 22, particularly preferably 10 - 18, most preferably 12 - 16 when the N-acylamino acid or a salt thereof is a mixture. When the acyl group chain length (carbon

atom number of acyl group (average carbon atom number)) is less than 8, an improving effect on the lathering performance is difficult to achieve and a frictional feeling is produced, which in turn degrades (the sense of) usability. When the acyl group chain length (carbon atom number of acyl group (average carbon atom number)) is greater than 22, moreover, lathering property is degraded and slyminess is produced, which in turn degrades (the sense of) usability.

In the case of N-acylamino acid salt, a salt containing, as a basic component, alkali metals such as sodium, potassium and the like, alkaline earth metals such as magnesium, calcium and the like, organic amines such as monoethanolamine, diethanolamine, triethanolamine, 2-amino-2-methyl-1-propanol, 2-amino-2-methyl-1,3-propanediol and the like, inorganic amines such as ammonia and the like, basic amino acids such as lysine, ornithine, arginine and the like, and the like can be mentioned. Alkali metal salts are preferable from the viewpoints of spreading of lather and lather retention.

N-acylamino acid or a salt thereof (excluding N-acylalanine or a salt thereof for the aforementioned (A)) for ingredient (D) may be used alone or two or more kinds thereof may be used concurrently. While the amount thereof to be added is not particularly limited, the weight ratio to ingredient (A) (D/A) is preferably 1/99-99/1, more preferably 10/90-90/10. When the amount of ingredient (D) is small and apart from the weight ratio, lather retention cannot be sufficiently improved and when the amount of ingredient (D) is high, problems such as insufficient lathering property, slimyness, frictional feeling and the like unpreferably occur easily. Thus, D/A is preferably within the range of 1/99-99/1 from the viewpoints of further improvement of lathering performance such as lather retention and the like, while maintaining a superior feel (sense of use) by ingredient (A).

The powder or granular cleansing composition of the present invention is generally used for cleansing agents for skin and/or hair, and other powder component can be added freely as long as it can be provided in a powder. Other components are, for example, cleansing base, moisturizer, excipient, anti-inflammatory agent, oil agent, coloring agent, flavor and the like.

The powder or granular cleansing composition of the present invention can be obtained by powderizing or granulating the above-mentioned various starting material components by known powderization (granulation) methods. To be specific, for example, various starting material components are mixed and pulverized, granulated·dried by fluidized granulation methods, and passed through a sieve to give the object powder or granular cleansing composition.

The powder or granular cleansing composition of the present invention can be used as a cleansing component of various hair cleansing agents such as shampoo, conditioning shampoo and the like, various skin cleansing agents such as face cleansing agents, makeup removing agents, cleansing cream, body shampoo, hand soap, solid soap, shaving foam, shaving cream and the like, and can be also used for toothpaste and the like.

The present invention is explained in more detail in the following by referring to Examples, which are not to be construed as limitative.

[Experiment 1]

**Example 1**

Water (929.25 g) was added to DL-alanine (166.15 g), and lauryl chloride (408 g) and 27 wt% aqueous NaOH solution were simultaneously added dropwise over 1.5 hr with stirring while maintaining pH of 10.98 - 11.02 and reaction temperature of 33 - 37°C. After the completion of the dropwise addition, the reaction mixture was heated to 50°C, aged for 3 hr, further heated to 85°C and 75 wt% sulfuric acid was added to adjust the pH to 2.0. The mixture was further stirred for about 10 min and stood still for 10 min to allow partitioning into an organic layer and an aqueous layer. The aqueous layer (about 1492 g) was removed from the bottom part of a container. Hot water (1000 g) was added to the remaining organic layer, and the mixture was heated again to 85°C, stirred for about 10 min and stood still for 10 min. The partitioned aqueous layer (904 g) was removed to give an organic layer. Water was removed from the obtained organic layer under reduced pressure and the layer was recrystallized from methanol to give N-lauroyl-DL-alanine. Then 48% aqueous potassium hydroxide solution and water were added to neutralize N-lauroyl-DL-alanine (neutralization equivalent amount 0.9) and adjusted to 25 wt% aqueous N-lauroyl-DL-alanine potassium salt solution. The obtained 25 wt% aqueous N-lauroyl-DL-alanine potassium salt solution was dried at 105°C for 1 hr and the resulting product was finely pulverized in a mortar to give lauroyl-DL-alanine potassium salt. The chain length distribution of the obtained lauroyl-DL-alanine potassium salt was measured by high performance liquid chromatography (HPLC) and acyl group chain length distribution was obtained from the peak area ratio.

**Example 2**

A necessary amount of potassium laurate was added to 25 wt% aqueous N-acylalanine salt solution obtained in Example 1, which was dried at 105°C for 1 hr and the obtained product was finely pulverized in a mortar to give lauroyl-DL-alanine potassium salt containing potassium laurate.

**Examples 3 - 8, Comparative Examples 1, 2**

In Examples 3 - 4, 7 and 8, N-acyl-DL-alanine salt shown in Table 1 were synthesized using single chain length acid chloride in the same manner as in Example 1. In addition, N-acyl-DL-alanine salts were synthesized in the same manner as in Example 1 and using NAA-312 chloride (manufactured by NOF Corporation, trade name) in Example 5, using NAA-415 chloride (manufactured by NOF Corporation, trade name) in Example 6, using acid chloride of coconut oil fatty acid (manufactured by NOF Corporation) in Comparative Example 1, and using acid chloride of coconut oil fatty acid (manufactured by Akzo) in Comparative Example 2. In Examples 4 - 8, moreover, the long chain fatty acid salt described in Table 1 was added in the amount defined therein to aqueous N-acyl-DL-alanine salt solution and the aqueous solution was dried, in the same manner as in Example 2.

Of the solid N-acyl-DL-alanine potassium salts prepared above in Examples 1 - 8, solid N-acyl-DL-alanine potassium salts produced in Examples 1 - 4 and 7 were measured for melting point, which was found to be 90°C, 88°C, 90°C, 83°C and 78°C, respectively.

The melting point measurement was performed using a differential scanning calorimeter (DSC) (DSC6200 manufactured by Seiko Instruments Inc.). For the measurement, the temperature was raised within the range of from 25°C to 200°C at 5°C/min and the peak top value of endothermic peak was taken as the melting point. When a main peak was not obtained up to 200°C, not less than 200°C was taken as the melting point.

Solid N-acyl-DL-alanine potassium salts prepared above in Examples 1 - 8 and Comparative Examples 1, 2 were evaluated as follows. The results are shown in the following Table 1.

1. Evaluation of producibility (release property evaluation)

A 25 wt% aqueous N-acylalanine salt solution was placed in an aluminum container, dried at 105°C for 1 hr, and cooled to room temperature, after which the properties of solid N-acylalanine salt were evaluated based on the following grade table.

⊙: solid free of stickiness and easily scratched off
O: solid somewhat sticky but easily scratched off
Δ: sticky solid which is rather difficult to scratch off
×: solid with pine resin-like stickiness and difficult to scratch off

2. Evaluation of moisture absorbability during preservation

The moisture absorbability was measured under the following measurement conditions and using an automatic vapor absorption measurement apparatus "BELSORP18" manufactured by BELL JAPAN, INC.

[measurement conditions]

- air thermostatic tank temperature: 50°C
- measurement temperature: 25°C
- MAX intake pressure: 0.9×Ps (Ps: saturated vapor pressure at measurement temperature)
- MIN exhaust pressure: 0.1×Ps (Ps: saturated vapor pressure at measurement temperature)
- equilibration time: 5 min

[Evaluation]

Average equilibrated moisture of less than 5% at humidity 70% RH is ⊙, not less than 5% - less than 10% is ○, not less than 10% - less than 15% is Δ, not less than 15% is x.

3. Evaluation of caking during preservation

For evaluation of caking, a powder sample (25 g) was placed in a cylinder having an inner diameter of 40 mm, a spindle (770 g) was placed thereon, the whole apparatus was placed in a zipper type plastic bag and preserved in a thermostatic tank (50°C) for 24 hr. The condition of the powder after 24 hr was visually observed and evaluated based on the following grade table.

x: caking was observed in the entirety
Δ: caking was observed in not less than half of powder
O: caking was scarcely observed
⊙: caking was not observed

4. Evaluation of solubility upon dissolution in water

For evaluation of solubility, an evaluation sample (0.2 g) was taken on a palm of the hand, tap water (2.0 g) was added, they were mixed 20 times with a finger and the state then was visually evaluated. Six professional panellers observed by the following evaluation criteria, and an average evaluation point of the 6 panellers was calculated.

The evaluation criteria were: not less than 1 - less than 2 is x, not less than 2.0 - less than 2.5 is Δ, not less than 2.5 - less than 3.0 is O and 3 is ⊙.

1: large agglomeration remains
2: somewhat undissolved
3: uniformly dissolved
5. Evaluation of powder recovery rate by spray dryer

30 wt% aqueous solutions of N-acylalanine salts described in Examples 1 - 8, 18 - 25 and Comparative Examples 1 - 2 were prepared, dried in a spray dryer (LT-8 type, manufactured by Okawara Mfg. Co., Ltd.) and the recovery rate of powder was determined. As regards Examples 1 - 8 and Comparative Examples 1 - 2 (potassium salt), the inlet temperature was 65°C, and as regards Examples 18 - 25 (arginine salt and lysine salt), the inlet temperature was 140°C. The powder recovery rate was calculated from the weight ratio of the obtained powder based on the amount of N-acylalanine salt present in the aqueous solutions before drying as 100%. Furthermore, the obtained recovery rate was evaluated based on the following grade table.

⊙: not less than 70%
○: not less than 60% - less than 70%
Δ: not less than 50% - less than 60%
x: less than 50%

Table 1

| Component name | Example 1 | Example 2 | Example 3 | Example 4 | Example 5 | Example 6 | Example 7 | Example 8 | Comparative Example 1 | Comparative Example 2 |
|---|---|---|---|---|---|---|---|---|---|---|
| potassium capryloyl-DL-alaninate | | | | | | 3.8 | | | 4.5 | 7.3 |
| potassium caproyl-DL-alaninate | | | 1.0 | 1.0 | 9.4 | 6.6 | 0.9 | 5.0 | 5.4 | 5.7 |
| potassium lauroyl-DL-alaninate | 100.0 | 98.0 | 98.0 | 96.0 | 70.5 | 56.4 | 88.1 | 65.0 | 43.2 | 45.0 |
| potassium myristoyl-DL-alaninate | | | 1.0 | 1.0 | 14.1 | 23.5 | 0.9 | 8.0 | 18.0 | 16.9 |
| potassium palmitoyl-DL-alaninate | | | | | | 2.8 | 0.1 | 2.0 | 9.0 | 7.7 |
| potassium stearoyl-DL-alaninate | | | | | | 0.9 | | | 9.9 | 7.4 |
| potassium laurate | | 2.0 | | 2.0 | 6.0 | 6.0 | 10.0 | 16.0 | 10.0 | 10.0 |
| potassium myristate | | | | | | | | 4.0 | | |
| acylalanine used | | | | | | | | | | |
| neutralization equivalent amount | 0.90 | 0.95 | 0.90 | 0.90 | 0.95 | 1.00 | 0.90 | 1.00 | 0.95 | 0.95 |
| standard deviation (σ) of acyl group | 0.00 | 0.00 | 0.28 | 0.29 | 1.00 | 1.62 | 0.31 | 1.01 | 2.48 | 2.47 |
| evaluation results | | | | | | | | | | |
| release property after drying | ○ | ⊙ | ○ | ⊙ | ○ | ○ | ○ | Δ | × | × |
| moisture absorbability during preservation | ⊙ | ⊙ | ⊙ | ⊙ | ○ | Δ | ⊙ | ○ | × | × |
| caking during preservation | ⊙ | ⊙ | ⊙ | ⊙ | ○ | Δ | ○ | ○ | Δ | Δ |
| solubility | ○ | ⊙ | ○ | ⊙ | ⊙ | ○ | ○ | Δ | × | × |
| powder recovery amount | ○ | ○ | ○ | ○ | Δ | Δ | Δ | Δ | × | × |

From Table 1, it is clear that N-acylalanine potassium salt can be superior in solubility and anti-caking property when the standard deviation of acyl group chain length distribution is not more than 2.0.

**Examples 18 - 25**

In the same manner as in Example 1 except that acid chlorides having different chain lengths were mixed and used, N-acylalanine was obtained. In Examples 18 - 24, neutralization was performed using L-arginine to achieve the neutralization equivalent amounts described in Table 2. In Example 25, neutralization was performed using L-lysine to achieve the neutralization equivalent amount described in Table 2.

In Example 18, the aqueous solution obtained above was added and in Examples 19 - 25, fatty acid in the amounts described in Table 2 was added, and they were respectively dried.

Of the solid N-acyl-DL-alanine arginine salts and lysine salt prepared above in Examples 18 - 25, the melting point of the salts of Examples 18 - 24 was measured and found to be not less than 200°C for all of them.

The solid N-acyl-DL-alaninearginine salts and lysine salt of Examples 18 - 25 were evaluated in the same manner as in the aforementioned Examples 1 - 7 and Comparative Examples 1, 2. The results are shown in the following Table 2..

Table 2

| | Example | | | | | | | |
|---|---|---|---|---|---|---|---|---|
| Component name | 18 | 19 | 20 | 21 | 22 | 23 | 24 | 25 |
| arginine capryloyl-DL-alaninate | | | 3.8 | 3.6 | 4.5 | | 14.2 | |
| arginine caproyl-DL-alanine | 1.0 | 9.5 | 7.7 | 7.2 | 5.4 | 14.01 | 14.2 | |
| arginine lauroyl-DL-alanine | 98.0 | 71.3 | 57.6 | 54.0 | 43.2 | 23.2 | 4.8 | |
| arginine myristoyl-DL-alaninate | 1.0 | 14.2 | 24.0 | 22.5 | 18.0 | 9.3 | 4.8 | |
| arginine palmitoyl-DL-alaninate | | | 2.9 | 2.7 | 9.0 | 9.3 | 19.0 | |
| arginine stearoyl-DL-alaninate | | | | | 9.9 | 37.2 | 38.0 | |
| lysine capryloyl-DL-alaninate | | | | | | | | 3.8 |
| lysine caproyl-DL-alaninate | | | | | | | | 7.7 |
| lysine lauroyl-DL-alaninate | | | | | | | | 57.6 |
| lysine myristoyl-DL-alaninate | | | | | | | | 24.0 |
| lysine palmitoyl-DL-alaninate | | | | | | | | 2.9 |
| arginine laurate | | 5.0 | 2.8 | 7.0 | 7.0 | 4.0 | 3.5 | |
| arginine myristate | | | 1.2 | 3.0 | 3.0 | 3.0 | 1.5 | |
| lysine laurate | | | | | | | | 2.8 |
| lysine myristate | | | | | | | | 1.2 |
| acylalanine used | | | | | | | | |
| neutralization equivalent amount | 0.95 | 0.90 | 0.95 | 1.00 | 0.95 | 0.95 | 1.00 | 0.95 |
| standard deviation (σ) of acyl group | 0.28 | 1.00 | 1.54 | 1.54 | 2.48 | 3.13 | 3.90 | 1.54 |
| evaluation results | | | | | | | | |
| release property after drying | ⊙ | ⊙ | ⊙ | ⊙ | ⊙ | ⊙ | ⊙ | ⊙ |
| moisture absorbability during preservation | ○ | ○ | ○ | ○ | ○ | ○ | ○ | ○ |
| caking during preservation | ⊙ | O | O | O | O | O | ○ | O |
| solubility | ⊙ | ⊙ | ⊙ | ⊙ | ⊙ | ⊙ | ⊙ | ⊙ |
| powder recovery amount | ⊙ | ⊙ | ⊙ | ⊙ | ⊙ | ⊙ | ⊙ | ⊙ |

From Table 2, it is clear that the basic amino acid salt of N-acylalanine has high powder recovery rate as

compared to potassium salt, and powderization is possible in a wider range of standard deviation of acyl group chain length distribution than that of potassium salt. In the case of a basic amino acid salt, moreover, it is superior in solubility at any chain length distribution.

[Experiment 2]

**Examples 9 - 17, 26 - 27 and Comparative Examples 3 - 6**

The respective components (materials) having the formulations shown in the following Table 3 were mixed and pulverized to give powder cleansing compositions, which were subjected to the following evaluation tests.

6. Evaluation of caking during preservation

The measurement apparatus and measurement conditions were the same as in the aforementioned 3.

7. Evaluation of solubility

An evaluation sample (0.2 g) was taken on a palm of the hand, tap water (2.0 g) was added, they were mixed 20 times with a finger and the state then was visually evaluated. Six professional panellers observed by the following evaluation criteria, and an average evaluation point of the 6 panellers was calculated. The evaluation criteria were: not less than 1 - less than 2 is x, not less than 2.0 - less than 2.5 is Δ, not less than 2.5 - less than 3.0 is O and 3 is ⊙.

1: large agglomeration remains
2: somewhat undissolved
3: uniformly dissolved

8. Evaluation of speed of lathering, lather amount, lather retention and slimyness and frictional feeling after use

Functional evaluation by face washing using an evaluation sample was performed. The functional evaluation was made by 6 professional panellers according to the following evaluation criteria and the average point of 6 panellers was calculated. The evaluation criteria were: not less than 1 - less than 2 is ×, not less than 2 - less than 3 is Δ, not less than 3 - less than 4 is O, and 4 is ⊙.

- speed of lathering

  1: late
  2: rather late
  3: rather quick
  4: quick

- lather amount

  1: insufficient
  2: rather insufficient
  3: rather thick
  4: thick

- lather retention

  1: weak
  2: rather weak
  3: rather strong
  4: strong

- slimyness after use

  1: slimyness is felt
  2: slimyness is somewhat felt

3: slimyness is scarcely felt
4: slimyness is not felt

- frictional feeling after use

  1: friction is felt
  2: friction is somewhat felt
  3: friction is scarcely felt
  4: friction is not felt

Table 3

| | Examples | | | | | | | | | | | Com. Examples | | | |
|---|---|---|---|---|---|---|---|---|---|---|---|---|---|---|---|
| Name of ingredient | 9 | 10 | 11 | 12 | 13 | 14 | 15 | 16 | 17 | 26 | 27 | 3 | 4 | 5 | 6 |
| solid acylalanine salt of Ex. 1 | 80.0 | 40.0 | 40.0 | 30.0 | 18.0 | | | | | | | | | | |
| solid acylalanine salt of Ex. 4 | | | | | | 30.0 | 30.0 | | | | | | | | |
| solid acylalanine salt of Ex. 5 | | | | | | | | 30.0 | | | | | | | |
| solid acylalanine salt of Ex. 6 | | | | | | | | | 30.0 | | | | | | |
| solid acylalanine salt of Ex. 18 | | | | | | | | | | 30.0 | | | | | |
| solid acylalanine salt of Ex. 20 | | | | | | | | | | | 30.0 | | | | |
| solid acylalanine salt of Com. Ex. 1 | | | | | | | | | | | | 30.0 | | | |
| solid acylalanine salt of Com. Ex. 2 | | | | | | | | | | | | | 30.0 | | |
| sodium lauroyl-L-grutamate *1 | - | - | - | - | 10.0 | - | 5.0 | 3.0 | 3.0 | 10.0 | 10.0 | - | 5.0 | 2.0 | 20.0 |
| sodium cocoyl glycinate *2 | - | - | - | - | 12.0 | - | - | 2.0 | 2.0 | 12.0 | 12.0 | - | 3.0 | - | 10.0 |
| sodium cocoyl isethionate *3 | - | - | - | 10.0 | - | - | - | 1.0 | 1.0 | 1.0 | 1.0 | - | 10.0 | 10.0 | 20.0 |
| potassium laurate *4 | - | - | 0.5 | 0.5 | 0.5 | 0.5 | 0.5 | 0.1 | 0.1 | 0.1 | 0.1 | 0.5 | 0.5 | 0.2 | 0.5 |
| D-mannitol | 20.0 | 20.0 | 20.0 | 20.0 | 20.0 | 20.0 | 15.0 | 15.0 | 15.0 | 20.0 | 20.0 | 20.0 | 20.0 | 30.0 | 20.0 |
| proline | - | - | - | - | - | - | - | 0.1 | 0.1 | 0.1 | 0.1 | 0.1 | 0.1 | 0.1 | - |
| serine | - | - | - | - | - | - | 0.2 | - | - | 0.2 | 0.2 | - | - | - | 0.1 |
| talc | - | 10.0 | 9.5 | 9.5 | 9.5 | 29.5 | 29.3 | 28.8 | 28.8 | 6.6 | 6.6 | 29.4 | 19.4 | 19.7 | 27.4 |
| cornstarch | - | 30.0 | 30.0 | 30.0 | 30.0 | 20.0 | 20.0 | 20.0 | 20.0 | 20.0 | 20.0 | 20.0 | 20.0 | 20.0 | 20.0 |

| | acylalanine used | | | | | | | | | | | | | | |
|---|---|---|---|---|---|---|---|---|---|---|---|---|---|---|---|
| neutralization equivalent amount | 0.90 | 0.90 | 0.90 | 0.90 | 0.90 | 0.90 | 0.90 | 0.95 | 0.95 | 0.95 | 0.95 | 0.90 | 0.90 | - | - |
| standard deviation (σ) of acyl group | 0.00 | 0.00 | 0.00 | 0.00 | 0.00 | 0.29 | 0.29 | 1.00 | 1.62 | 0.28 | 1.54 | 2.48 | 2.47 | - | - |
| | evaluation results | | | | | | | | | | | | | | |
| caking during preservation | ○ | ◎ | ◎ | ◎ | ◎ | ◎ | ◎ | Δ | Δ | ○ | ○ | × | × | ◎ | ◎ |
| solubility in practical use | ○ | ○ | ◎ | ◎ | ◎ | ◎ | ◎ | ○ | ○ | ◎ | ◎ | × | × | ○ | ○ |
| quick lathering | ◎ | ◎ | ◎ | ◎ | ○ | ◎ | ○ | ○ | ○ | ◎ | ◎ | Δ | Δ | × | ○ |
| lather amount | ◎ | ◎ | ◎ | ◎ | ◎ | ◎ | ◎ | ○ | ○ | ◎ | ◎ | Δ | Δ | × | ◎ |
| lather retention | ○ | ○ | ○ | ◎ | ◎ | ◎ | ◎ | ○ | ○ | ○ | ◎ | ○ | ○ | × | ○ |
| slimyness during use | ◎ | ◎ | ◎ | ◎ | ◎ | ◎ | ○ | ○ | ○ | ◎ | ◎ | ○ | ○ | × | ○ |
| friction during use | ◎ | ◎ | ◎ | ◎ | ◎ | ◎ | ◎ | ◎ | ◎ | ◎ | ◎ | ○ | ○ | ○ | × |

*1: "Amisoft" LS-11 (manufactured by AJINOMOTO CO., INC.)

*2: "Amilite" GCS-11 (manufactured by AJINOMOTO CO., INC.)

*3: Jordapon CI-P (manufactured by BASF)

*4: LK-2 (manufactured by NOF Corporation)

**Formulation Examples 1 - 8**

The powder cleansing compositions having the formulations shown in the following Table 4 - Table 11 were produced according to conventional methods. All compositions were free of caking due to moisture absorption during preservation, and agglomeration of powder in practical use. Moreover, they were fast in lathering, superior in the basic performance of cleansing agents such as lather amount and lather retention, free of slimyness and friction during use, and safe with less irritation.

Table 4 Formulation Example 1 (powder cleansing composition)

| substances added | amount added (wt%) |
|---|---|
| solid acylalanine salt of Ex. 4 | 20.0 |
| sodium lauroyl glutamate *1) | 5.0 |
| sodium cocoyl glycinate *2) | 2.0 |
| sodium cocoyl isethionate *3) | 1.0 |
| sodium myristate | 0.5 |
| D-mannitol | 40.0 |
| proline | 0.2 |
| talc | 15.0 |
| cornstarch | 16.0 |
| allantoin | 0.1 |
| lauroyl lysine *4) | 0.2 |
| total | 100 |

Table 5 Formulation Example 2 (powder cleansing composition)

| substances added | amount added (wt%) |
|---|---|
| solid acylalanine salt of Ex. 5 | 5.0 |
| sodium lauroyl glutamate *1) | 8.0 |
| sodium myristoyl glutamate *5) | 4.0 |
| sodium palmoyl glutamate *6) | 2.0 |
| sodium cocoyl glycinate *2) | 2.0 |
| sodium cocoyl isethionate *3) | 1.0 |
| D-mannitol | 43.0 |
| glycine | 0.2 |
| talc | 15.0 |
| cornstarch | 19.0 |
| allantoin | 0.1 |
| lauroyl lysine | 0.4 |
| crystalline cellulose | 0.2 |
| polyquaternium-10 | 0.1 |
| total | 100.0 |

Table 6 Formulation Example 3 (powder cleansing composition)

| substances added | amount added (wt%) |
|---|---|
| potassium caproyl-L-alaninate | 2.8 |
| potassium lauroyl-L-alaninate | 21.5 |
| potassium myristoyl-L-alaninate | 4.2 |
| potassium laurate | 0.2 |
| sodium lauroyl glutamate *1) | 4.0 |
| sodium myristoyl glutamate *5) | 2.0 |
| sodium cocoyl isethionate *3) | 3.0 |
| D-mannitol | 30.0 |
| serine | 0.4 |
| talc | 18.0 |
| cornstarch | 13.3 |
| lauroyl lysine | 0.3 |
| dextrin | 0.1 |
| polyquaternium-10 | 0.2 |
| total | 100.0 |
| standard deviation of acyl group chain length distribution of acylalanine salt: 0.99 | |

Table 7 Formulation Example 4 (powder cleansing composition)

| substances added | amount added (wt%) |
|---|---|
| potassium caproyl-DL-alaninate | 0.1 |
| potassium lauroyl-DL-alaninate | 9.8 |
| potassium myristoyl-DL-alaninate | 0.1 |
| sodium lauroyl glutamate *1) | 8.0 |
| sodium myristoyl glutamate *5) | 2.0 |
| sodium cocoyl glycinate *2) | 10.0 |
| D-mannitol | 40.0 |
| serine | 0.1 |
| talc | 20.0 |
| cornstarch | 9.9 |
| total | 100.0 |
| standard deviation of acyl group chain length distribution of acylalanine salt: 0.28 | |

Table 8 Formulation Example 5 (powder cleansing composition)

| substances added | amount added (wt%) |
|---|---|
| potassium capryloyl-L-alaninate | 0.4 |
| potassium caproyl-L-alaninate | 0.7 |
| potassium lauroyl-L-alaninate | 5.6 |

Table continued

| substances added | amount added (wt%) |
|---|---|
| potassium myristoyl-L-alaninate | 2.4 |
| potassium palmitoyl-L-alaninate | 0.3 |
| potassium stearoyl-L-alaninate | 0.1 |
| sodium lauroyl glutamate *1) | 15.0 |
| sodium myristoyl glutamate *5) | 3.0 |
| sodium cocoyl glycinate *2) | 4.0 |
| potassium laurate | 3.0 |
| sodium myristate | 1.0 |
| D-mannitol | 20.0 |
| serine | 0.1 |
| talc | 15.0 |
| cornstarch | 28.9 |
| lauroyl lysine | 0.5 |
| total | 100.0 |
| standard deviation of acyl group chain length distribution of acylalanine salt: 1.66 | |

Table 9 Formulation Example 6 (powder cleansing composition)

| substances added | amount added (wt%) |
|---|---|
| potassium capryloyl-L-alaninate | 0.6 |
| potassium caproyl-L-alaninate | 1.0 |
| potassium lauroyl-L-alaninate | 8.5 |
| potassium myristoyl-L-alaninate | 3.5 |
| potassium palmitoyl-L-alaninate | 0.4 |
| potassium stearoyl-L-alaninate | 0.1 |
| sodium lauroyl glutamate *1) | 2.0 |
| sodium myristoyl glutamate *5) | 1.0 |
| sodium cocoyl glycinate *2) | 1.0 |
| potassium laurate | 3.0 |
| sodium myristate | 3.0 |
| D-mannitol | 30.0 |
| serine | 0.4 |
| talc | 20.0 |
| cornstarch | 25.2 |
| lauroyl lysine | 0.2 |
| polyquaternium-10 | 0.1 |
| total | 100.0 |
| standard deviation of acyl group chain length distribution of acylalanine salt: 1.61 | |

Table 10 Formulation Example 7 (powder cleansing composition)

| substances added | amount added (wt%) |
|---|---|
| solid acylalanine salt described in Example 21 | 20.0 |
| sodium lauroyl glutamate *1) | 5.0 |
| sodium cocoyl glycinate *2) | 2.0 |
| sodium cocoyl isethionate *3) | 1.0 |
| sodium myristate | 0.5 |
| D-mannitol | 40.0 |
| proline | 0.2 |
| talc | 15.0 |
| cornstarch | 16.0 |
| allantoin | 0.1 |
| lauroyl lysine *4) | 0.2 |
| total | 100.0 |

Table 11 Formulation Example 8 (powder cleansing composition)

| substances added | amount added (wt%) |
|---|---|
| solid acylalanine salt described in Example 19 | 5.0 |
| sodium lauroyl glutamate *1) | 8.0 |
| sodium myristoyl glutamate *5) | 4.0 |
| sodium palmoyl glutamate *6) | 2.0 |
| sodium cocoyl glycinate *2) | 2.0 |
| sodium cocoyl isethionate *3) | 1.0 |
| D-mannitol | 43.0 |
| glycine | 0.2 |
| talc | 15.0 |
| cornstarch | 19.0 |
| allantoin | 0.1 |
| lauroyl lysine | 0.4 |
| crystalline cellulose | 0.2 |
| polyquaternium-10 | 0.1 |
| total | 100.0 |

In Tables 4 - 11, *1)-*6) show the following compounds.
*1) "Amisoft" LS-11 (manufactured by Ajinomoto Co., Inc.)
*2) "Amilite" GCS-11 (manufactured by Ajinomoto Co., Inc.)
*3) Jordapon CI-P (manufactured by BASF)
*4) "Amihope" LL (manufactured by Ajinomoto Co., Inc.)
*5) "Amisoft" MS-11 (manufactured by Ajinomoto Co., Inc.)
*6) "Amisoft" GS-11P (manufactured by Ajinomoto Co., Inc.)

## Industrial Applicability

As is clear from the foregoing explanation, according to the present invention, solid N-acylalanine and/or a salt thereof which are/is easily solidified, and which are/is low moisture absorptive and superior in solubility in water can be obtained, and the solid N-acylalanine and/or a salt thereof can be preferably used as cleansing components for various forms of cleansing agents. Particularly, since a powder or granular cleansing composition containing the solid N-acylalanine and/or a salt thereof is free of caking due to moisture absorption during preservation and agglomeration of powder in practical use, and which is quick in lathering, superior in the basic performance of cleansing agents such as lather amount and lather retention, free of slimyness and friction during use, and safe with less irritation, cleansing agents for skin and/or hair having extremely high property can be realized.

This application is based on application No. 2003-129626 filed in Japan, the contents of which are incorporated hereinto by reference.

## Claims

1. A solid N-acylalanine comprising one kind or two or more kinds of compounds, each having an acyl group having a different number of carbon atoms, which is/are selected from the compounds represented by the formula (I):

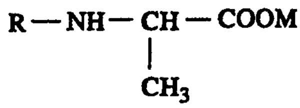

wherein R is a straight chain or branched chain, saturated or unsaturated acyl group having 8 to 22 carbon atoms, and M is a hydrogen atom, an alkali metal, an alkaline earth metal, ammonium, an organic amine or a basic amino acid, or a salt thereof.

2. The solid N-acylalanine of claim 1, which comprises a long chain fatty acid or a salt thereof, or a salt thereof.

3. A solid N-acylalanine comprising one kind or two or more kinds of compounds, each having an acyl group having a different number of carbon atoms, which is/are selected from the compounds represented by the formula (I):

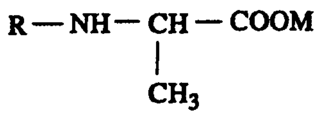

wherein R is a straight chain or branched chain, saturated or unsaturated acyl group having 8 to 22 carbon atoms, and M is a hydrogen atom, an alkali metal, an alkaline earth metal, ammonium or an organic amine,
which shows the standard deviation ($\sigma$) of the acyl group chain length distribution of not more than 2.0,
or a salt thereof.

4. The solid N-acylalanine of claim 3, wherein M in the formula (I) is an alkali metal, or a salt thereof.

5. The solid N-acylalanine of claim 4, wherein the alkali metal is potassium, or a salt thereof.

6. The solid N-acylalanine of any one of claims 3 - 5, which comprises a long chain fatty acid or a salt thereof, or a salt thereof.

7. A solid N-acylalanine comprising one kind or two or more kinds of compounds, each having an acyl group having a different number of carbon atoms, which is/are selected from the compounds represented by the formula (I):

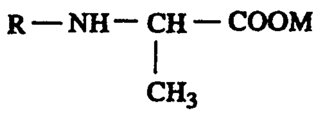

wherein R is a straight chain or branched chain, saturated or unsaturated acyl group having 8 to 22 carbon atoms, and M is a basic amino acid,
which shows the standard deviation (σ) of the acyl group chain length distribution of not more than 5.0,
or a salt thereof.

**8.** The solid N-acylalanine of claim 7, which comprises a long chain fatty acid or a salt thereof, or a salt thereof.

**9.** The solid N-acylalanine of claim 7 or 8, wherein the basic amino acid for M in the formula (I) is lysine or arginine, or a salt thereof.

**10.** The solid N-acylalanine of any one of claims 1 - 9, which is used for a powder or granular cleansing agent, or a salt thereof.

**11.** A powder or granular cleansing composition comprising at least (A) the solid N-acylalanine of any one of claims 1 - 9 or a salt thereof.

**12.** The powder or granular cleansing composition of claim 11, further comprising (B) an inorganic or organic powder.

**13.** The powder or granular cleansing composition of claim 11 or 12, further comprising (C) acylisethionic acid or a salt thereof and/or (D) acylamino acid or a salt thereof (excluding the N-acylalanine or a salt thereof for the aforementioned (A)).

**14.** A cleansing agent for skin or hair, comprising the powder or granular cleansing composition of any one of claims 11 - 13.

**INTERNATIONAL SEARCH REPORT**

International application No.
PCT/JP2004/006114

A. CLASSIFICATION OF SUBJECT MATTER
Int.Cl[7] C07C233/47, A61K7/075, 7/50

According to International Patent Classification (IPC) or to both national classification and IPC

B. FIELDS SEARCHED

Minimum documentation searched (classification system followed by classification symbols)
Int.Cl[7] C07C233/47, A61K7/075, 7/50

Documentation searched other than minimum documentation to the extent that such documents are included in the fields searched

Electronic data base consulted during the international search (name of data base and, where practicable, search terms used)
CAPLUS(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 9-13078 A (Ajinomoto Co., Inc.), 14 January, 1997 (14.01.97), (Family: none) | 1-14 |
| A | JP 63-2962 A (Kawaken Fine Chemicals Co., Ltd.), 07 January, 1988 (07.01.88), (Family: none) | 1-14 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 June, 2004 (02.06.04) | 22 June, 2004 (22.06.04) |
| Name and mailing address of the ISA/ Japanese Patent Office | Authorized officer |
| Facsimile No. | Telephone No. |

Form PCT/ISA/210 (second sheet) (January 2004)